(19) Europäisches Patentamt

European Patent Office

Office européen des brevets

(11) Veröffentlichungsnummer : **0 236 393 B1**

(12) **EUROPÄISCHE PATENTSCHRIFT**

(45) Veröffentlichungstag der Patentschrift :
**11.03.92 Patentblatt 92/11**

(51) Int. Cl.[5] : **C12M 1/02, C12M 1/40, C12P 7/06**

(21) Anmeldenummer : **86905292.8**

(22) Anmeldetag : **18.09.86**

(86) Internationale Anmeldenummer :
**PCT/EP86/00539**

(87) Internationale Veröffentlichungsnummer :
**WO 87/01725 26.03.87 Gazette 87/07**

(54) **BIOTECHNOLOGISCHES KONTINUIERLICHES VERFAHREN ZUR HYDROLYSE VON KOHLENHYDRATEN UND DIE SIMULTANE WEITERVERARBEITUNG DER SPALTPRODUKTE DURCH MIKROORGANISMEN.**

(30) Priorität : **19.09.85 DE 3533352**

(43) Veröffentlichungstag der Anmeldung :
**16.09.87 Patentblatt 87/38**

(45) Bekanntmachung des Hinweises auf die
Patenterteilung :
**11.03.92 Patentblatt 92/11**

(84) Benannte Vertragsstaaten :
**AT BE CH DE FR GB IT LI LU NL SE**

(56) Entgegenhaltungen :
**EP-A- 0 142 230
Cereal chemistry, volume 61, n 5, September/october 1984 (St Paul, US), J.E. McGhee et aö. "Continuous bioconversion of starch to ethanol by calcium-alginate immobilized enzymes and yeasts", pages 446-449, see chapter "Materials and methods", page 446
International sugar journal, volume 82, n 974, february 1980 (High Wycombe, Bucks, GB), "Continuous alcohol fermentation in Australia", pages 44-45
Chemical abstracts, volume 102, n 3, 21 January 1985 (Colombus, Ohio, US) L.H. Wang et al. "Studies on ethanol production by immobilized xylose isomerase and active yeast cells", page 543, abstract n 22771d, T'ai-wan T'ang Yeh Yen chiu, So Yen Chiu Hui Pao 1983, 100
Biotechnology & Bioengineering, volume 27, n 1,5,7,9,10, 1985 (New York, US), W.K. Jain et al. "Continuous production of ethanol from fructose by immobilized growing cells of zymomonas mobilis", pages 613-620**

(56) Entgegenhaltungen :
**Chemical abstracts, volume 97, n 21, 22 November 1982. (Colombus, Ohio, US), I.G. Prince et al. "Tower fermentation using Zymomonas mobilis for ethanol production", page 625
Chemical abstracts, volume 97, n 23, 6 December 1982 (Colombus, Ohio, US), page 454, abstract n 196822q, & JP A 82110192 (Mitsubishi Chemical Industries Co, Ltd), 8 July 1982
Biotechnology letters, Band 11, n 8, 1982, pp. 525-530
See also references of WO8701725**

(73) Patentinhaber : **Kraftanlagen AG.
Im Breitspiel 7
W-6900 Heidelberg 1 (DE)**

(72) Erfinder : **TRAMM-WERNER, Sabine
Schurzelter Strasse 208
W-5100 Aachen (DE)**
Erfinder : **HARTMEIER, Winfried
Melatener Strasse 145a
W-5100 Aachen (DE)**

(74) Vertreter : **Helber, Friedrich G., Dipl.-Ing. et al
Zenz, Helber & Hosbach Patentanwälte
Scheuergasse 24
W-6144 Zwingenberg (DE)**

EP 0 236 393 B1

**Beschreibung**

Die Erfindung betrifft ein biotechnologisches, kontinuierliches Verfahren für die Hydrolyse nicht metabolisierbarer Kohlenhydrate durch Enzyme und die simultane Weiterverarbeitung der Spaltprodukte durch Mikroorganismen zu Ethanol, unter Immobilisierung der Mikroorganismen und der Enzyme.

Zur Herstellung von Ethanol aus Stärke unter Einsatz von immobilisierten Hefen und immobilisierten Enzymen wurden bereits Versuche durchgeführt (Cerreal Chemistry, Band 61, Nr. 5, 1984, Seiten 446 -449). Für das einstufige Verfahren wurde Natriumalginat mit Hefe und Glucoseamylase, sowie destilliertem Wasser vermischt. Die Alginatmischung tropfenweise abgezogen und zur Bildung von Kalziumalginatkapseln, diese in eine kalziumchloridverstärkte Eintragslösung eingebracht. Gegenstand dieser Versuche ist die gleichzeitige Immobilisierung von Mikroorganismen (Hefen) und von Enzymen (Glycoamylasen) durch ihren Einschluß in eine feste Matrix, und damit ihre unmittelbare Verbindung. Die Matrix vergrößert als inaktives Material das Reaktionsvolumen, nimmt zugleich die entstehenden Reaktionsgase auf, schwimmt dadurch an die Oberfläche auf, wodurch schließlich Probleme bei der Abtrennung des Produkts an der Oberfläche auftreten. Die verwendete Matrix erfordert eine Sonderbehandlung der Mikroorganismen.

Zur Herstellung von Ethanol aus D-cylose wurden desweiteren immobilisierte aktive Hefezellen als Mikroorganismen gemeisam mit dem Enzym Cyloseisomerase in einer zweistufigen, d.h. mehrstufigen Verfahrensführung innerhalb in Reihe geschalteter Rohrreaktoren eingesetzt (Chemical Abstracts, Band 102, Nr. 3, 1985, Seite 543, Zusammenfassung Nr. 2271d). Das Substrat wird mehrmals im Kreislauf geführt. Die mehrfache Produktrückführung birgt die Gefahr einer Infektion der Mikroorganismen.

Auch in diesem Fall wird eine positive Immobilisierung eingesetzt. Hefezellen werden an den Innenwandungen bzw. innerhalb der Wandungen von Hohlfasern oder Rohren nach Art von Membranen zurückgehalten. Das Substrat diffundiert durch Membranwände der Hohlfasern in denen die Hefezellen eingeschlossen sind. Die Durchflußrate durch den Reaktor steht in direkter Abhängigkeit zur Wachstumsrate der Mikroorganismen.

Ein in Australien untersuchtes kontinuierliches Gäverfahren zur Ethanolherstellung setzt als Substrat gleichfalls kohlenhydrathaltige Lösungen, Zuckerrohrsaft oder Melasse aus der Zuckerherstellung ein (International Sugar Journal, Band 82, 1982, Seiten 525 - 530). Die Verfahrensführung erfolgt aerob unter Einsatz von Hefen als Mikroorganismen. Das Substrat wurde hierbei im Gleichstrom mit dem Sauerstoff geführt. Innerhalb des Fermentationsturms folgen ein Abschnitt zur Einmischung des Sauerstoff in den Substratstrom, ein weiterer Abschnitt zur Rückhaltung der Hefe unter wahlweiser Beheizung oder Kühlung, ein dritter Abschnitt zur wahlweisen Nachheizung oder Kühlung und schließlich ein letzter Abschnitt zur Beruhigung für ein Ausscheiden des Kohlendioxids, sowie die Abtrennung des Produkts aufeinander. Als Hinderungsgrund für eine erhöhte Alkoholproduktion wird auf eine Hefemutation und niedrige Gärgeschwindigkeiten (Fermentationsraten) hingewiesen. Die Verfahrensführung erfolgt unter aeroben Bedingungen. Zur Herstellung von Fructose und Ethanol aus Rohr- oder Rübenzucker durch eine alkoholische Gärung ist es gleichfalls bekannt dem Substrat als Mikroorganismen Zymomonas mobilis und/oder immmobilisierte Levansucrase als Fermentationsmittel, Enzym zuzusetzen (EP-A- 142 230). Dem Substrat wird zunächst Levansucrase zugegeben, über einen Zeitraum von 24 bis 30 Stunden die Einwirkung, Inkubation vorgenommen, danach die Mikroorganismen eingeleitet und das Substrat durch Mikroorganismen vergoren. Das Produkt wird desweiteren von den Mikroorganismen und/oder den Enzymen durch Filter oder Zentrifugen abgetrennt.

Es handelt sich um einen ansatzweisen Batch-Betrieb. Als Biokatalysator dient immobilisierte Levansucrase.

Schließlich sind Untersuchungen zur Vergärung von Glucose zu Ethanol unter Einsatz geflockter Zymomonas mobilis bekannt (Biotechnology Letters, Band 4, Nr. 8, 1982, Seiten 525 - 530). Die Mikroorganismen wurden in einem Kulturmedium bezogen auf 100 gr. Substrat Glucose mit 1 gr. $KH_2PO_4$, 1 gr. $(NH_4)_2 SO_4$, 0,5 gr. $MgSO_4 \times 7 H_2O$ und 5 gr. Hefeextrakt vorgezogen und nachfolgend durch Filtration sterilisiert.

Nach den vorbekannten Verfahren werden die unterschiedlichsten Produkte unter Einsatz immobilisierter Mikroorganismen vorallem Hefen und gleichfalls immobilisierter Enzyme hergestellt. Kennzeichnend für diese Verfahren ist die Verwendung einer Matrix zur Immobilisierung der Mikroorganismen nach Art einer Matrix-Verknüpfung vorallem durch Einschluß in oder durch oberflächliches Aufbringen auf eine feste Matrix in den unterschiedlichsten Formgebungen. Eine derartige Matrix erfordert eine besondere Abstimmung auf die verwendeten Mikroorganismen, desweiteren Maßnahmen gegen den Eintrag von Zellgiften mit dieser Matrix sowie Maßnahmen gegen eine Anreicherung eingetragener Zellgifte innerhalb derselben im Verlauf des Herstellungsprozesses.

Um Mikroorganismen Zugang zu für sie nicht oder nur schwer verfügbaren Substraten zu verschaffen, wurden bisher mehrere Methoden angewendet:

1. Mehrstufige Verfahren; dabei wird in der ersten Stufe das Substrat für den entsprechenden Organismus enzymatisch aufgearbeitet, in den folgenden Schritten erfolgt dann der mikrobielle Abbau (übliches Verfahren der Kornbrennereien, z.B. A.P.V. Company Ltd., Crawley, West Sussex RH 10 2QB, England).

2. Versuche, die fehlenden enzymatischen Eingenschaften genetisch eizukreuzen, um eine einstufige Prozeßführung zu ermöglichen (Goodman A E, Strzelecki A T, Rogers P L (1984): Formation of ethanol from lactose by zymomonas mobilis, J Biotechnol 1, 219-228).

3. Verschiedene Arten von Coimmobilisierung von Mikroorganismen mit Enzymen (Hartmeier, W (1985): Immobilized biocatalysts -from simple to complex systems, Trends in Biotechnol 3, 149 - 153), bei denen Enzyme durch chemische oder physikalische Methoden mit den Mikroorganismen verknüpft werden und im Reaktionsraum zurückgehalten werden.

Mehrstufige Aufschlußverfahren haben den Nachteil, daß sie relativ zeitaufwendig sind, da die erste umsetzung abgeschlossen sein muß, bevor mit der zweiten Stufe begonnen werden kann. Außerdem sind bei enzymatischen Umsetzungen sogenannte Endprodukthemmugen häufig, so daß eine vollständige Substratumsetzung nur schwer zu erreichen ist.

Im industriellen Maßstab kommt es bei Arbeiten mit genetisch veränderten Organismen häufig zu Rückmutationen, die die gewünschte Eingenschaft nicht mehr besitzen.

Die Coimmobilisierung von Organismen mit den jeweils erwünschten Enzymen führt zu mehr oder weniger starken Aktivitätsverlusten der Organismen, da sie bei der Herstellung Kontakt mit Zellgiften, z.B. Glutaraldehyd, bekommen, oder auch mit einer zusätzlichen Diffusionsbarriere umgeben werden.

Zur Steigerung der volumetrischen Produktivität ist es vorteilhaft, die Zelldichte im Reaktor zu erhöhen. Dazu gibt es grundsätzlich folgende Möglichkeiten.

1. Immobilisierung der Zellen (Vorlop, KD, Klein, J (1982): New developments in the field of Cell immobilization - Formation of Biocatalyst by ionotropic gelation, in: Enzyme Technology, III Rotenburg fermentation Symposium 1982; Lafferty R M, Maier E (eds.) Springer Verlag Berlin, Heiderlberg, New York, Tokio 1983)

2. Arbeiten mit Separatoren (Biostill[R], Alfa Laval).

3. Einsatz von Filtermembranen (Starcosa GmbH, Braunschweig)

4. Einsatz von flockenden Organismen in Turmreaktoren (Strandberg G W, Donaldson T L, Arcuri E J, (1982): Continuous ethanol production by a flocculent strain of Zymomonas mobilis, Biotechnol lett 4, 347 - 352).

Die Verfahren 1 bis 3 haben den Nachteil, daß neben den erwünschten Organismen auch Fremdkeime in den Reaktionsraum zurückgeführt und angereichert werden. Der Einsatz von Filtermembranen kann im technischen langzeitbetrieb problematisch sein, da sich die Membrane leicht zusetzt.

Beim Gebrauch von Turmreaktoren treten diese Nachteil nicht auf, da nicht flokende Fremdkeime mit dem Produktstrom augeschwemmt werden. Bisher gebräuchliche Turmreaktoren mit aufgesetztem Separator haben aber nicht die doppelte Wirkung durch a) Verlangsamung der Strömungsgeschwindigkeit durch einen verbreiterten Durchmesser und gleichzeitig b) den eingesetzten Zylinder oder oben offenen Kegel zur Schaffung einer turbulenzfreien Außenzone mit Produktüberlauf.

Der Erfindung liegt die Aufgabe zugrunde, die langen Ketten der Kohlenhydrate in kürzere Bruchstücke aufzuspalten um diese Bruchstücke dem Stoffwechsel der Mikroorganismen zugänglich zu machen und sie zu Ethanol abzubauen.

Die Aufgabe wird erfindungsgemäß dadurch gelöst, daß anaerob flockende Zymomonas mobilis als Mikroorganismen eingesetzt werden, den physikalischen Eingenschaften der Mikroorganismenflocken, diejenigen von Hydrolasen als Enzyme durch deren Immobilisierung angepaßt werden, daß desweiteren Mikroorgansimen und Enzyme gleichzeitig in enen Turmreaktor eingeführt werden, ferner daß diesem Turmreaktor das Substrat kontinuierlich von unten zugeleitet und diesem das Produkt über einen ringzylindrischen Überlauf eines erweiterten oberen Endstücks kontinuierlich entnommen wird. Der Reaktor hat ein erweitertes oberes Endstück, wodurch die Fließgeschwindigkeit reduziert ist. Im erweiterten Endstück befindet sich ein eingesetzter Zylinder, dessen Durchmesser geringfügig größer ist als der Innendurchmesser des Reaktors (siehe Zeichnung). Im Inneren des Zylinders steigen die bei der Reaktion entstehenden Gase auf, während sich außerhalb eine turbulenzfreie Zone befindet, in der der Überlauf angeordnet ist. Durch diese Konstruktion bleibt der Produktstrom frei von Bakterien- und Enzymflocken, und die Bakterien reichern sich im Reaktor an. Es können Durchflußraten errreicht werden, die höher sind als die Wachstumsraten der meisten Organismen, so daß sich nur flockende Organismen im Reaktor halten können, während nichtflockende Fremdkeime mit dem Produktstrom augeschwemmt werden.

Als Substrate kommen kohlenhydrathaltige Flüssigmedien mit keinem oder nur geringem Feststoffanteil in Frage, wie zum Beispiel Molke oder Lactosesirup, Melasse, Zuckerrübensaft, inulinhaltige Sirupe, Stärkehydrolysate etc.

Vorteilhaft werden Mischungen unterschiedlicher, kohlenhydrathaltiger Substrate eingesetzt.

Besonders vorteilhaft sind Mischungen aus verdünnter Melasse und/oder milchzuckerhaltigem Sirup, vorzugsweise mit einem Gesamtzuckergehalt von mindestens 14% als Substrat. Milchzucker ist in hohen Konzentration durch Lactase nur schwer vollständig abbaubar, da das Enzym durch die bei der Reaktion entstehende Galactose hemmbar ist. Hohe Zuckerkonzentrationen im Substrat sind aber wünschenswert, um die Produktkonzentration zu erhöhen. Da wegen der hohen Salzkonzentrationen von Melasse auch hier eine bestimmte Zuckerkonzentration nicht überschritten werden kann, bietet sich eine Mischung von verdünnter Melasse mit Milchzucker an. Dadurch wird bei hoher Gesamtzuckerkonzentration die Salzbelastung durch die Melasse reduziert und die Milchzuckerkonzentration kann reduziert werden.

Vorteilhaft sind auch mehrere Enzyme gleichzeitig zu verwenden; auch hierdurch ist eine höhere Substratkonzentration einzusetzen, ohne das Risiko von Produkthemmungen einzugehen. Diesbezüglich zur Fermentation vorteilhaft sind immobilisierte Invertase in Mischung mit gleichfalls immobilisierter Lactase. Gleichfalls vorteilhaft ist es flockende Zymomonas mobilis in einem Nährmedium aus Kaliumhydrogenphosphat, Amonium- und Magnesiumsulfat, sowie Yeast-Extrakt vorzuziehen, Amylasen als Enzyme zur Immobilisierung mit destilliertem Wasser und Albumin zunächst zu mischen, anschließend mittels Aceton zu fällen und unter Zusatz von Glutaraldehyd in wässriger Lösung zu inkubieren und schließlich mehrmals zu waschen, desweiteren zur Fermentation die vorgezogenen, flockenden Zymomonas mobilis und die immobilisierten Amylasen in einen Turmreaktor einzubringen und diesen das zuckerhaltige Substrat kontinuierlich von unten zuzuführen sowie das Produkt Ethanol laufend oben zu entnehmen.

Desweiteren vorteilhaft werden hierbei zur Fermentation immobilisierte Zymomonas mobilis und Amyloglucosidase in den Reaktor eingeführt, 10% Maltodextrin, 0,1% Magnesiumsulfat x7 $H_2O$, 0,1% Amoniumsulfat und 0,1% Kaliumdihydrogenphosphat und 0,5% Yerst-Extrakt als Substrat kontinuierlich zugegeben, eine Temperatur von 30°C konstant gehalten, durch Zugabe einer einmolaren Natronlauge ein pH-Wert von ca. 4,5 eingestellt, sowie aufrechterhalten und kontinuierlich Ethanol abgezogen.

Ein vorteilhafter Weg der Anpassung der physikalischen Eigenschaften der immobilisierten Enzyme an diejenigen der Mikroorganismenflocken besteht darin, daß lysinreiche Eiweiße, vorzugsweise Albumin, zugesetzt werden.

Das Beispiel erläutert die Erfindung:

Vergärung von Maltodextrin zu Ethanol mit immobilisierter Amyloglucosidase und einem flockenden Stamm von Zymomonas mobilis.

Bakterienvorzucht: Die Bakterien werden 24 Stunden in 1l Nährmedium mit 1g $MgSO_4$x7$H_2O$, 1g Ammonsulfat, 1g Kaliumdihydrogenphosphat, 5g Yeast Extract und 100g Glucose bei 28° C vorgezogen.

Enzymimmobilisierung: Für ein Reaktorvolumen von 700ml werden 21g Gluczyme (Amano), 150ml destilliertes Wasser und 1,4g Albumin gemischt, im Eisbad mit 300ml Aceton gefällt und mit 10ml Glutaraldehyd (25%ig, wäßrig) 2 Stunden bei 25°C inkubiert. Anschließend wird dreimal mit Wasser gewaschen.

Fermentation: Immobilisiertes Enzyme und Bakterien werden in den Turmreaktor (siehe Abbildung) überführt. Als Substrat wird Maltodextrin in oben angegebenem Medium mit 10% Maltodextrin (statt Glucose) verwendet. Bei einer Durchflußrate von 0,214 erhält man im Produktstrom 48g/l Ethanol, 2,9 g/l Glucose und 1,8g/l Maltodextrin.

Während der Fermentation wird die Temperatur auf 30°C gehalten und der pH-Wert mit Natronlauge (1molar) auf pH 4,5 gehalten.

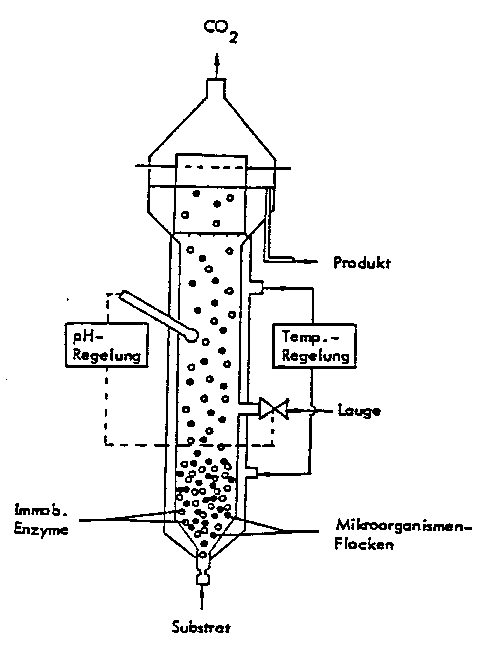

## Patentansprüche

1. Biotechnologisches, kontinuierliches Verfahren für die Hydrolyse nicht metabolisierbarer Kohlenhydrate durch Enzyme und die simultane Weiterverarbeitung der Spaltprodukte durch Mikroorganismen zu Ethanol unter Immobilisierung der Mikroorganismen und der Enzyme dadurch gekennzeichnet, daß anaerob flockende Zymomonas mobilis als Mikroorganismen eingesetzt werden, den physikalischen Eigenschaften der Mikroorganismenflocken, diejenigen von Hydrolasen als Enzyme durch deren Immobilisierung angepaßt werden, daß desweiteren Mikroorganismen und Enzyme gleichzeitig in einem Trumreaktor eingeführt werden, ferner daß diesem Turmreaktor das Substrat kontinuierlich von unten zugeleitet und diesem das Produkt über einen ringzylindrischen Oberlauf eines erweiterten oberen Endstück kontinuierlich entnommen wird.

2. Verfahren nach Anspruch 1, dadurch gekennzeichnet, daß Mischungen unterschiedlicher, kohlenhydrathaltiger Substrate eingesetzt werden.

3. Verfahren nach Anspruch 2, dadurch gekennzeichnet, daß eine Mischung aus verdünnter Melasse und-/oder milchzuckerhaltigem Sirup, vorzugsweise mit einem Gesamtzuckergehalt von mindestens 14%, als Substrat eingesetzt wird.

4. Verfahren nach Anspruch 3, dadurch gekennzeichnet, daß gleichzeitig verschiedene immobilisierte Enzyme zugesetzt werden.

5. Verfahren nach Anspruch 4, dadurch gekennzeichnet, daß zur Fermentation immobilisierte Invertase in Mischung mit gleichfalls immobilisierter Lactase zugesetzt wird.

6. Verfahren nach Anspruch 1, 3, dadurch gekennzeichnet, daß flockende Zymomonas mobilis in einem Nährmedium aus Kaliumhydrogenphosphat, Amonium- und Magnesiumsulfat, sowie Yeast-Extrakt vorgezogen, Amylasen als Enzyme zur Immobilisierung mit destilliertem Wasser und Albumin zunächst gemischt, anschließend mittels Aceton gefällt und unter Zusatz von Glutaraldehyd in wässriger Lösung inkubiert und schließlich mehrmals gewaschen werden, daß desweiteren zur Fermentation die vorgezogenen flockenden Zymomonas mobilis und die immobilisierten Amylasen in einen Turmreaktor eingebracht werden, und diesen das zuckerhaltige Substrat kontinuierlich von unten zugeführt und das Produkt Ethanol laufend oben entnommen wird.

7. Verfahren nach Anspruch 6, dadurch gekennzeichnet, daß zur Fermentation immobilisierte Zymomonas mobilis und Amyloglucosidase in den Reaktor eingeführt, 10% Maltodextrin, 0,1% Magnesiumsulfat 7 $H_2O$, 0,1% Amoniumsulfat und 0,1% Kaliumdihydrogenphosphat und 0,5% Yeast-Extrakt als Substrat kontinuierlich zugegeben, eine Temperatur von 30°C konstant gehalten durch Zugabe einer einmolaren Natronlauge ein pH-Wert von ca. 4,5 eingestellt, sowie aufrechterhalten und kontinuierlich Ethanol abgezogen.

8. Verfahren nach Anspruch 1, dadurch gekennzeichnet, daß die physikalischen Eigenschaften der immobilisierten Enzyme denjenigen der Mikroorganismenflocken durch Zusatz lysinreicher Eiweiße, vorzugsweise Albumin, angepaßt werden.

## Claims

1. Biotechnological, continuous process for the hydrolysis of nonmetabolizable carbohydrates by enzymes and the simultaneous processing of the cleavage products to ethanol with immobilization of the microorganisms and enzymes, characterized in that anaerobically flocculent Zymomonas mobilis are used as microorganisms, the physical properties of the flocculent microorganisms and enzymes are adapted to those of hydrolases as enzymes by their immobilization, that furthermore microorganisms and enzymes are introduced simultaneously into a tower reactor, furthermore that the substrate is fed continuously to this tower reactor from below, and from the tower the product is removed continuously over a cylindrical weir of an expanded upper end piece.

2. Process according to claim 1, characterized in that mixture of different, carbohydrate -containing substrates are used.

3. Process according to claim 2, characterized in that a mixture of dilute molasses and/or syrup containing milk sugar, preferably with a total sugar content of at least 14%, is used as substrate.

4. Process according to claim 3, characterized in that different immobilized enzymes are added simultaneously.

5. Process according to claim 4, characterized in that invertase immobilized for the fermentation is added in mixture with likewise immobilized lactase.

6. Process according to claims 1 and 3, characterized in that flocculent Zymomonas mobilis is precultured in a nutrient medium of potassium hydrogen phosphate, ammonium sulfate and magnesium sulfate as well as yeast extract, amylases as enzymes for the immobilization are first mixed with distilled water and albumin, then precipitated with acetone, and incubated in aqueous solution with the addition of glutaraldehyde, and finally repeatedly washed, that furthermore for the fermentation the precultured flocculent Zymomonas mobilis and the immobilized amylases are put into a tower reactor, and the sugar -containing substrate is fed to the latter continuously from below and the product ethanol is continuously removed at the top.

7. Process according to claim 6, characterized in that Zymomonas mobilis and amyloglucosidase immobilized for the fermentation are introduced into the reactor, 10% maltodextrin, 0.1% magnesium sulfate 7 $H_2O$, 0.1% ammonium sulfate and 0.1% potassium dihydrogen phosphate and 0.5% yeast extract are continuously added as substrate, a temperature of 30°C is held constant by the addition of a monomolar soda lye, a pH of approximately 4.5 is established and sustained, and ethanol is withdrawn continuously.

8. Process according to claim 1, characterized in that the physical properties of the immobilized enzymes are adapted to those of the flocculent microorganisms by the addition of lysine -rich proteins, preferably albumin.

## Revendications

1. Procédé continu biotechnologique pour l'hydrolyse d'hydrates de carbone non métabolisables par des enzymes et le traitement ultérieur simultané des produits de dissociation par des microorganismes pour obtenir de l'éthanol, avec immobilisation des microorganismes et des enzymes, caractérisé par le fait que l'on utilise, comme microorganismes, Zymomonas mobilis floculant en l'absence d'air, on adapte aux propriétés physiques des flocons des microorganismes celles d'hydrolases en tant qu'enzymes en immobilisant celles-ci, en outre on introduit simultanément des microorganismes et des enzymes dans un réacteur vertical, on amène le substrat en continu au réacteur vertical par le bas et on en prélève le produit en continu par un trop-plein annulaire cylindrique d'une pièce d'extrémité supérieure évasée.

2. Procédé selon la revendication 1, caractérisé par le fait que l'on utilise des mélanges de différents substrats contenant des hydrates de carbone.

3. Procédé selon la revendication 2, caractérisé par le fait que l'on utilise comme substrat un mélange de mélasse diluée et/ou de sirop contenant du lactose, de préférence d'une teneur totale en sucre d'au moins 14 %.

4. Procédé selon la revendication 3, caractérisé par le fait que l'on ajoute simultanément différentes enzymes immobilisées.

5. Procédé selon la revendication 4, caractérisé par le fait que l'on ajoute, pour la fermentation, de l'invertase immobilisée en mélange avec de la lactase également immobilisée.

6. Procédé selon la revendication 1, 3, caractérisé par le fait que l'on cultive d'abord Zymomonas mobilis floculant dans un milieu de culture composé de phosphate acide de potassium, sulfate d'ammonium et sulfate de magnésium, ainsi que d'extrait d'Yeast, on mélange des amylases, en tant qu'enzymes, d'abord avec de l'eau distillée et de l'albumine en vue de leur immobilisation, puis on les précipite au moyen d'acétone et les met à incuber en solution aqueuse avec addition de glutaraldéhyde et, enfin, on les lave à plusieurs reprises, en outre, en vue de la fermentation, on introduit dans un réacteur vertical Zymomonas mobilis floculant, cultivé préalablement, ainsi que les amylases immobilisées, on amène aux amylases, en continu, par le bas, le substrat contenant du sucre et on prélève en continu, par le haut, le produit éthanol.

7. Procédé selon la revendication 6, caractérisé par le fait qu'en vue de la fermentation, on introduit dans le réacteur Zymomonas mobilis immobilisé et de l'amyloglucosidase, on ajoute en continu, en tant que substrat, 10 % de maltodextrine, 0,1 % de sulfate de magnésium 7 $H_2O$, 0,1 % de sulfate d'ammonium et 0,1 % de dihydrogénophosphate de potassium, ainsi que 0,5 % d'extrait d'Yeast, on maintient constamment une température de 30 degrés C, on règle à pH d'environ 4,5 et maintient à cette valeur par addition d'une lessive de soude 1-molaire et on évacue en continu l'éthanol.

8. Procédé selon la revendication 1, caractérisé par le fait que les propriétés physiques des enzymes immobilisées sont adaptées à celles des flocons de microorganismes par addition d'albumines riches en lysine, de préférence d'albumine.

CO$_2$

Produkt

pH-Regelung

Temp.-Regelung

Lauge

Immob. Enzyme

Mikroorganismen-Flocken

Substrat